# EUROPEAN PATENT APPLICATION

(11) **EP 2 223 617 A1**
(43) Date of publication of application: **01.09.2010**
(21) Application number: 08876726.4
(22) Date of filing: 20.11.2008
(51) Int. Cl.: A23L 2/52, A23L 1/302, A23L 1/30

(54) **METHOD FOR PRODUCING A SUBSTANCE FOR REDUCING ALCOHOL LEVELS IN THE BLOOD, AND SUBSTANCE PRODUCE**

(71) Applicant: Platinum Prestige, S.L., 03202 Eiche (Alicante) (ES)
(72) Inventor: BELDA NICOLAS, Jose Antonio, E-03206 Alicante, (ES)
(74) Representative: De la Fuente Fernandez, Dionisio
(86) International application number: PCT/ES2008/000728
(87) International publication number: WO 2010/043726

(57) **Abstract**

The invention describes a method for the production of a substance effective for reducing blood alcohol levels resulting from the excessive consumption of alcoholic beverages, and it also describes the substance itself. The method consists of preparing a combination obtained by mixing in a stainless steel mixing drum a group of natural ingredients including crystalline fructose, glucose syrup, citric acid, vitamin C, vitamins B1, B6 and B15, fluid extracts of ginseng and guarana, potassium sorbate and liquid lemon flavor; the mixing is performed for a time of 15-20 minutes at ambient temperature, and vials are filled with the obtained combination. The end substance has properties for the enhancement of the purifying power, the partial inhibition of alcohol absorption at the digestive tract level, and the neutralization of the desire to consume alcohol.

## Description

### Object of the Invention

The present invention relates to a method for the manufacture of a substance for reducing blood alcohol levels, which provides essential novelty features and remarkable advantages with respect to the means known and used for the same purposes in the current state of the art. Likewise, the invention also relates to the substance obtained by means of the mentioned method, and to the use thereof in the reduction of blood alcohol levels.

According to the foregoing, the invention specifically proposes the development of a method by means of which a substance which aids in eliminating alcohol in the organism is obtained, with the particularity of simultaneously reducing the need for consuming alcohol experienced by the person concerned. The substance is prepared based on natural ingredients, free of other health risks, and is a powerful adjuvant for eliminating alcohol by virtue of its behavior under a triple effect for the organism: enhancing the purifying power when there is a presence of alcohol in the blood, partially preventing the absorption of the alcohol consumed at digestive tract level, and neutralizing the desire to drink alcohol.

The field of application of the invention obviously belongs to the industrial sector dedicated to the preparation and/or manufacture of products and substances especially indicated against the harmful effects resulting from the consumption of alcohol.

### Background and Summary of the Invention

As is generally known, the consumption of alcoholic beverages has multiple harmful effects on the health and general behavior of people who consume them. Alcoholic beverages, which have a wide diversity of presentations and alcohol content in the current state of the art, contain ethyl alcohol (ethanol) and cause dependence (alcoholism) in people who consume them uncontrollably, entailing multiple health risks together with atypical, sometimes aggressive, behavior which normally rejected from a social point of view.

More specifically, from the health point of view, ethanol is a potent psychoactive drug causing intoxication the effects of which have a greater or lesser duration depending on the amount of alcohol which is consumed, and has a biphasic effect which consists of a sensation of relaxation and happiness at first, and of coordination problems or even blurred vision later. This is explained based on cell membrane permeability with respect to alcohol, so that once consumed, the bloodstream aids in its distribution through most of the body tissues. Even in extreme cases, when excessive consumption occurs, it can lead to a state of unconsciousness, to poisoning and finally to death of the person who has consumed this abusive amount of alcohol.

These states resulting from the abusive consumption of alcoholic beverages can also lead, as has been mentioned, to other situations, such as altered behavior of the individual, poor job performance, or slower reflexes in situations which require a fast reaction in order to prevent potential dangers, as the case of the behavior of a person behind the steering wheel of a vehicle. In fact, statistics show that alcohol is present in a very high percentage of work-related accidents, but especially the numbers of traffic accidents resulting in death occurring every year caused directly by the consumption of alcoholic beverages are truly staggering. In Spain, for example, the percentage of traffic accidents resulting from driving under the influence of alcohol is comprised between 30 and 50% in accidents with deaths, between 15 and 35% in accidents with serious injuries, and around 10% in accidents with mild injuries. In fact, it is recognized that in Spain, for example, about 63% of vehicle drivers are frequent drinkers (i.e., they consume alcoholic beverages at least once a week), whereas about 19% consume a daily mean of absolute alcohol of the order of 80 or more grams.

As will be understood, the consumption of alcohol above certain predefined levels can lead to many types of adverse situations of immeasurable consequences, therefore it would be desirable to provide some type of remedy which, if needed, could be applied to a person who must be rescued from an undesirable situation in which he or she has ended up inadvertently or because of habits.

Therefore, according to the indicated need, the present invention has proposed a main objective of producing a substance which allows solving the problem considered. An additional object of the invention consists of providing a substance which has no health risk for the person who uses it, and which is highly effective concerning the fast action for a significant reduction of the blood alcohol level.

According to these premises, the substance of the invention acts as a coadjuvant for eliminating alcohol present in people's organism, activating and enhancing the purifying mechanisms of the organism when the presence of alcohol in the blood is detected, while at the same time partially inhibiting the absorption of the consumed alcohol through the digestive tract, thereby facilitating its elimination and, together with neutralizing the desire of the individual to consume alcohol, making the substance especially suitable for solving this type of situation and making it especially useful for returning the individual to full awareness.

The preparation of the substance entails mixing a multitude of natural ingredients in precisely measured amounts for the subsequent packaging thereof in vials under controlled environmental conditions of asepsis and sterility. The different ingredients intervening in the formulation of the substance for reducing blood alcohol levels according to the invention have been especially selected for the sought purposes, although their action in the substance must be considered from a synergistic point of view, without the effect of each individual ingredient being significant for evaluating the end product as a whole.

A substance prepared for meeting objectives similar to those proposed by the present invention is known in the current state of the art, which substance can be consumed by a person in the event that blood alcohol levels exceed levels which must be necessarily reduced, and the mechanism of action of which consists of aiding to metabolize the alcohol level for its quicker elimination.

### Detailed Description of the Preferred Embodiment

As has been described above, the main objective of the present invention is the development of a method intended for the production of a substance that is especially suitable for its use in the quick, effective and safe reduction of the blood alcohol level of a person as a result of the consumption of alcoholic beverages, the level of which is considered as exceeding the levels considered normal, either from the health point of view, or from a functional point of view for performing a job activity or another type of activity. According to a preferred embodiment of the invention, the substance for reducing blood alcohol level consists of a combination of ingredients mixed in predefined proportions according to the qualitative and quantitative formula which is indicated below:

| | |
|---|---|
| Crystalline fructose | 2000 mg |
| Glucose syrup | 400 mg |
| Citric acid monohydrate | 25 mg |
| Vitamin C (calcium ascorbate) | 9 mg |
| Vitamin B1 (thiamine hydrochloride) | 2.5 mg |
| Vitamin B6 (pyridoxine hydrochloride) | 5 mg |
| Vitamin B15 (pangamic acid) | 25 mg |
| Potassium sorbate | 20 mg |
| Liquid lemon flavor | 3 mg |
| Fluid extract of ginseng | 300 mg |
| Fluid extract of guarana | 1000 mg |

According to the invention, the method for the preparation of a substance of this type includes the following steps:
1) A liquid mixing drum, particularly a conventional stainless drum, is initially prepared for performing a cold mixture at an ambient temperature comprised between approximately 18 and 20°C;
2) Then, after actuating the drum and making the propeller contained therein rotate, a specific amount of purified water, of the order of 10 ml, is added to which precise and previously weighed amounts of each of the aforementioned ingredients are successively added in the same order in which they have been specified;
3) The operation of mixing is maintained for a time of about 15-20 minutes in the mentioned conditions, after which the obtained combination is transferred to an intermediate stainless steel tank;
4) The substance is absorbed from this intermediate tank by means of volumetric pumps for its introduction in vials which, once filled, are conveyed by means of a belt on which they are capped and led to the exit for their placement in plastic supports.

For the operation of filling the vials the use of a suitable conventional automatic machine is preferred, for example of the type that are marketed under the Struck® trademark, although logically other conventional machines enabled for carrying out these types of operations with assurances for optimizing the obtained results could be used.

### Properties of the substance thus formulated

As has been previously mentioned, the substance for reducing blood alcohol levels obtained by means of the combination of ingredients formulated as has been specified in the foregoing, meets the envisaged objectives of enhancing the purifying power of the organism when it detects the presence of alcohol in the blood, partially preventing the absorption of alcohol at the digestive tract level thereby aiding its elimination, and furthermore neutralizing the desire to consume alcohol. This is possible due to the properties inherent to the components themselves which intervene in the combination, optimally mixed with one another, and the following comments are made as to the activity of some of such components:

### a. Vitamins B1, B6, B12

These components enhance the effect of vitamin B15. It is known that the consumption of alcohol destroys the reserves of B-complex vitamins; therefore the substance of the invention aids in recovering the levels of these vitamins.

Vitamin B1, or thiamine, plays an important role in the metabolism of carbohydrates (including alcohol), mainly to produce energy, and it furthermore participates in the metabolism of fats, proteins and nucleic acids. It is essential for the normal growth and development, and it aids in maintaining proper working of the heart, nervous system and digestive system. Thiamine is water-soluble, and the reserve in the body is low, being concentrated mainly in the skeletal muscle, presented as 80% TDP and 10% TTP, and the rest as free thiamine. Recent studies have shown that the consumption of foods rich in this vitamin aids in preventing certain serious effects of diabetes given that thiamine protects cells against high glucose levels.

Vitamin B6, or pyridoxine, intervenes in the preparation of mood-regulating substances in the brain, for example serotonin, forming an important aid for people who suffer depression, stress and sleep disorders. This vitamin, which, on the other hand, is very popular among athletes, increments muscle performance and energy production; this is because it favors the release of the glycogen stored in the liver and in the muscles when a greater effort needs to be performed. It also collaborates in reducing body weight because it aids in obtaining energy from accumulated fats.

Vitamin B12 is in turn present in a wide variety of foods.

It is water-soluble, often used in combination with other B-group vitamins and necessary for keeping neurons and red blood cells healthy, as well as for producing DNA, the genetic material of all cells. Once it is released from food digestion, it combines with a substance known as "intrinsic factor" before being absorbed by the bloodstream.

Clinical trials have shown that vitamin B12 deficiency can cause abnormal neurological and psychiatric symptoms such as ataxia, muscle weakness, spasticity, incontinence, hypotension, vision problems, dementia, psychosis, and mood disorders. According to the studies conducted, these symptoms can present at vitamin B12 levels that are simply somewhat lower than those considered as normal, whereas levels that are considerably higher than normal levels can give rise to anemias.

### b. Vitamin C

This vitamin enhances likewise the effect of vitamin B15.

### c. Pangamic acid or vitamin B15

This is a substance present in a number of plants. Minimum daily requirements of this substance have not yet been established as has occurred with the remaining vitamins, although it has certain similarities with vitamin E due to its antioxidant action, and it is water soluble. Its activity increases when it is used in combination with other B group vitamins. Its main sources are brewer's yeast, brown rice, whole grains, pumpkin seeds, and sesame seeds. It is currently not well known how it is absorbed and stored by the human body, but the excess amounts of this substance seem to be eliminated in urine, feces and breath.

The specific functionalities associated with this vitamin are the following:
- Neutralizes the desire to drink alcohol
- Protects against polluting agents
- Accelerates the recovery from fatigue
- Alleviates the symptoms of angina pectoris and asthma
- Prevents hangover
- It is present in protein synthesis
- Protects the liver from cirrhosis
- Extends cell life
- Reduces cholesterol levels
- Improves endocrine and nervous system functioning, and
- Improves blood circulation.

### d. Fluid extract of ginseng

One of the main effects observed as a detoxifier in this product consists of its anti-free radical action, i.e., as a free radical scavenger. Furthermore, it has been observed that it provides a protective effect against hepatotoxicity induced by alcohol and drugs, favoring the reduction of the blood concentration alcohol by virtue of reducing absorption at the digestive tract level provided by it. Preliminary studies indicate that ginseng can have protective effects on the liver, and it aids in eliminating blood alcohol levels.

### e. Fluid extract of guarana

This product is a physical and mental stimulant. It aids in regulating cardiovascular and gastrointestinal problems, gases, varices, arteriosclerosis, depressions, severe headaches, migraines, respiratory allergies, cholesterol, and it also oxygenates the blood and aids in reducing fever. It is effective against hangover (it is advisable to take it before drinking alcohol and the next day), and it is a very useful adaptogenic tonic in periods of fatigue.

### f. Fructose and lactose syrup

Even though these products are basically added as preservatives, they also accelerate the absorption at the intestinal level given that they are sugars which are very quickly absorbed.

As will be understand, the substance for reducing blood alcohol levels obtained from these ingredients is an effective remedy against the consequences of an excessive consumption of alcoholic beverages, without harmful side effects for the person who uses it, and in turn provides a restorative substance with very beneficial effects. These features, together with the neutralizing effect on the desire to consume alcohol, makes the substance of the invention especially suitable for counteracting the states resulting from a more or less abusive consumption of said alcoholic beverages.

Although the qualitative and quantitative formulation of the substance object of the invention has been described in detail in the foregoing, persons skilled in the art will understand that the latter must be understood for the purpose of illustrating and by no means limiting the invention, since the proportions and the ingredients will be susceptible of alterations and changes depending on the features that the end product is to have in each case.

## Claims

1. A method for the manufacture of a substance for reducing blood alcohol levels, particularly a method for the production of a substance which acts to quickly and effectively lower blood alcohol levels resulting from an excessive consumption of alcoholic beverages, which is **characterized in that** it comprises:
preparing a liquid mixing drum provided with an inner
propeller, particularly a mixing drum made of stainless steel, at a temperature for performing a cold mixture, particularly at an ambient temperature of the order of 18-20°C;
pouring a predefined amount of purified water into the mixing drum;
actuating the mixing drum, making the inner propeller rotate while the ingredients intervening in the composition of the substance for reducing blood alcohol level are added to said drum with the purified water therein, said ingredients being added in precisely measured amounts, and following a pre-established order;
mixing for a time period of the order of 15-20 minutes;
then transferring the mixture to an intermediate stainless steel tank;
filling vials with said mixture, with the use of volumetric pumps, and
placing the vials thus filled on a conveyor belt where they are capped and led to the exit for their collection and placement on plastic supports.

2. The method according to claim 1, **characterized in that** the ingredients intervening in step of addition into the mixing drum consist of:
Crystalline fructose,
Glucose syrup,
Citric acid monohydrate,
Vitamin C (calcium ascorbate),
Vitamin B1 (thiamine hydrochloride),
Vitamin B6 (pyridoxine hydrochloride),
Vitamin B15 (pangamic acid),
Potassium sorbate,
Liquid lemon flavor, and
Fluid extracts of plants (ginseng and guarana) successively introduced in the indicated order, and the amount of water previously introduced in the mixing drum being an amount of 10 ml.

3. A substance for reducing blood alcohol levels, especially suitable for quickly and effectively lower blood alcohol levels resulting from an excessive consumption of alcoholic beverages, obtained by means of the method of claim 1, **characterized in that** it consists of a combination of ingredients having the following qualitative and quantitative formula:
| | |
|---|---|
| Crystalline fructose | 2000 mg |
| Glucose syrup | 400 mg |
| Citric acid monohydrate | 25 mg |
| Vitamin C (calcium ascorbate) | 9 mg |
| Vitamin B1 (thiamine hydrochloride) | 2.5 mg |
| Vitamin B6 (pyridoxine hydrochloride) | 5 mg |
| Vitamin B15 (pangamic acid) | 25 mg |
| Potassium sorbate | 20 mg |
| Liquid lemon flavor | 3 mg |
| Fluid extract of ginseng | 300 mg |
| Fluid extract of guarana | 1000 mg |

4. The substance according to claim 3, **characterized in that** it has properties for the enhancement of the purifying power of the organism when it detects the presence of alcohol in the blood, the partial prevention of the absorption of alcohol consumed at the digestive tract level, and the neutralization of the desire to consume alcohol.

## Amended claims

### Amended claims under Art. 19.1 PCT

1. A method for the manufacture of a substance for reducing blood alcohol levels, particularly a method for the production of a substance which acts to quickly and effectively lower blood alcohol levels resulting from an excessive consumption of alcoholic beverages, which method comprises a series of steps consisting of:
preparing a liquid mixing drum provided with an inner propeller, especially made of stainless steel, at a temperature for performing a cold mixture, particularly at an ambient temperature of the order of 18-20°C;
pouring a predefined amount of purified water into the mixing drum;
actuating the mixing drum, making the inner propeller rotate while the ingredients intervening in the composition of the substance for reducing blood alcohol level are added to said drum with the purified water therein, said ingredients being added in precisely measured amounts, and following a pre-established order;
mixing the ingredients for a time period of the order of 15-20 minutes;
then transferring the mixture to an intermediate stainless steel tank;
filling vials with said mixture, with the use of volumetric pumps, and
placing the vials thus filled on a conveyor belt where they are capped and led to the exit for their collection and placement on plastic supports,
**characterized in that** the ingredients of the step of mixing are additional in precisely measured amounts, and consist of:
Crystalline fructose,
Glucose syrup,
Citric acid monohydrate,
Vitamin C (calcium ascorbate),
Vitamin B1 (thiamine hydrochloride),
Vitamin B6 (pyridoxine hydrochloride),
Vitamin B15 (pangamic acid),
Potassium sorbate,
Liquid lemon flavor, and
Fluid extracts of plants (ginseng and guarana) successively introduced in the indicated order, and the amount of water previously introduced in the mixing drum being an amount of about 10 ml.

2. A substance for reducing blood alcohol levels, especially suitable for quickly and effectively lowering blood alcohol levels resulting from an excessive consumption of alcoholic beverages, obtained by means of the method of claim 1, **characterized in that** it consists of a combination of ingredients having the following qualitative and quantitative formula:
| | |
|---|---|
| Crystalline fructose | 2000 mg |
| Glucose syrup | 400 mg |
| Citric acid monohydrate | 25 mg |
| Vitamin C (calcium ascorbate) | 9 mg |
| Vitamin B1 (thiamine hydrochloride) | 2.5 mg |
| Vitamin B6 (pyridoxine hydrochloride) | 5 mg |
| Vitamin B15 (pangamic acid) | 25 mg |
| Potassium sorbate | 20 mg |
| Liquid lemon flavor | 3 mg |
| Fluid extract of ginseng | 300 mg |
| Fluid extract of guarana | 1000 mg |

3. The substance according to claim 2, **characterized in that** it has properties for the enhancement of the purifying power of the organism when it detects the presence of alcohol in the blood, the partial prevention of the absorption of alcohol consumed at the digestive tract level, and the neutralization of the desire to consume alcohol.
